(19) Europäisches Patentamt
European Patent Office
Office européen des brevets

(11) Veröffentlichungsnummer: **0 011 770**
**B1**

# (12) EUROPÄISCHE PATENTSCHRIFT

(45) Veröffentlichungstag der Patentschrift: **14.10.81**

(21) Anmeldenummer: **79104485.2**

(22) Anmeldetag: **14.11.79**

(51) Int. Cl.³: **C 07 D 249/08,** C 07 D 233/60, A 61 K 31/41, A 61 K 31/415 // C07C33/46, C07C49/233

## (54) Fluorenyl-azolylmethyl-carbinole, Verfahren zu ihrer Herstellung, Arzneimittel, die diese Verbindungen enthalten und Verfahren zur Herstellung dieser Arzneimittel.

(30) Priorität: **25.11.78 DE 2851143**

(43) Veröffentlichungstag der Anmeldung: **11.06.80 Patentblatt 80/12**

(45) Bekanntmachung des Hinweises auf die Patenterteilung: **14.10.81 Patentblatt 81/41**

(84) Benannte Vertragsstaaten: **AT BE CH DE FR GB IT NL SE**

(56) Entgegenhaltungen: **DE-A-2 623 129**

(73) Patentinhaber: **BAYER AG, Zentralbereich Patente, Marken und Lizenzen, D-5090 Leverkusen 1, Bayerwerk (DE)**

(72) Erfinder: **Regel, Erik, Ing. grad., Bergerheide 72 a, D-5600 Wuppertal 1 (DE)**
Erfinder: **Büchel, Karl Heinz, Prof. Dr., Bergerheide 62, D-5600 Wuppertal 1 (DE)**
Erfinder: **Haller, Ingo, Dr., Viktoriastrasse 99, D-5600 Wuppertal 1 (DE)**
Erfinder: **Plempel, Manfred, Dr., Paikestrasse 5, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Fluorenyl-azolylmethyl-carbinole, Verfahren zu ihrer Herstellung, Arzneimittel, die diese Verbindungen enthalten und Verfahren zur Herstellung dieser Arzneimittel

Die vorliegende Erfindung betrifft neue Fluorenyl-azolylmethyl-carbinole, mehrere Verfahren zu ihrer Herstellung, Arzneimittel, insbesondere Antimykotika, die diese Verbindungen enthalten und ein Verfahren zur Herstellung dieser Arzneimittel.

Es ist bereits bekanntgeworden, daß 9-Azolyl-fluoren-Derivate gute antimykotische Wirkung aufweisen (vergleiche DE-A-2 004 697 und DE-A-2 053 080). Auch aus der DE-A-2 623 129 sind antimykotische Verbindungen bekanntgeworden, deren Wirkung gegen Dermatophyten jedoch ebenfalls nicht genügt. Deren Wirkung ist jedoch, insbesondere invivo gegen Dermatophyten, nicht immer ganz befriedigend.

Es wurden neue Fluorenyl-azolyl-methyl-carbinole der allgemeinen Formel

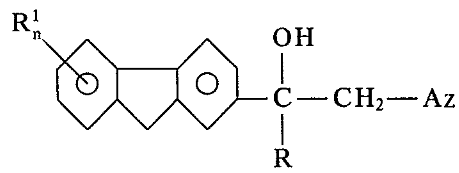

(I)

in welcher

Az für Imidazol-1-yl, 1,2,4-Triazol-1-yl oder 1,3,4-Triazol-1-yl steht,
R für gegebenenfalls durch Fluor oder Chlor ein- oder zweifach substituiertes Phenyl oder Benzyl steht,
$R^1$ für Fluor oder Chlor steht und
n für 0, 1 oder 2 steht,

und deren physiologisch verträglichen Säureadditions-Salze gefunden. Sie weisen starke antimykotische Eigenschaften auf und übertreffen in ihrer Wirkung gegen Dermatophyten diejenigen des genannten Standes der Technik.

Weiterhin wurde gefunden, daß man die Fluorenyl-azolyl-methyl-carbinole der Formel (I) erhält, wenn man

a) Fluorenyl-azolylmethyl-ketone der Formel

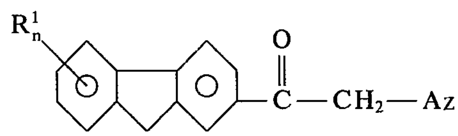

(II)

in welcher
Az, $R^1$ und n die oben angegebene Bedeutung haben,
mit einer Grignard-Verbindung der Formel

$$R-Mg-X \quad (III)$$

in welcher

R die oben angegebene Bedeutung hat und
X für Halogen, insbesondere Chlor oder Brom steht,

in Gegenwart eines Verdünnungsmittels umsetzt, oder

b) Fluorenyl-halogenmethyl-carbinole der Formel

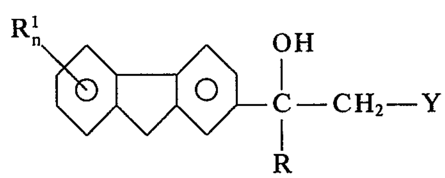

(IV)

in welcher

R, $R^1$ und n die oben angegebene Bedeutung haben und
Y für Halogen, insbesondere Chlor oder Brom steht,

mit Azolen der Formel

$$Z—Az \qquad (V)$$

in welcher

Az die oben angegebene Bedeutung hat und
Z für Wasserstoff oder ein Alkalimetall steht,

gegebenenfalls in Gegenwart eines Säurebindemittels und gegebenenfalls in Gegenwart eines Verdünnungsmittels umsetzt.

Weiterhin können die erfindungsgemäß erhältlichen Fluorenyl-azolyl-methyl-carbinole der Formel (I) durch Umsetzen mit Säuren in die Salze überführt werden.

Überraschenderweise zeigen die erfindungsgemäßen Fluorenyl-azolyl-methyl-carbinole eine bessere, therapeutisch nutzbare Wirksamkeit, insbesondere gegen Dermatophyten, als die aus dem Stand der Technik bekannten 9-Azolyl-fluoren-Derivate, welche chemisch und wirkungsmäßig naheliegende Verbindungen sind. Die Erfindungsgemäßen Wirkstoffe stellen somit eine wertvolle Bereicherung der Pharmazie dar.

Im einzelnen seien außer den bei den Herstellungsbeispielen genannten Verbindungen die folgenden Verbindungen der allgemeinen Formel (I) genannt:

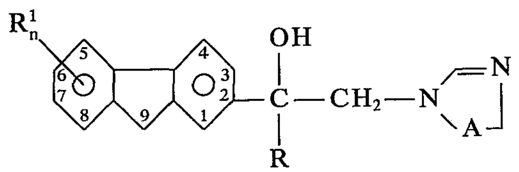

(Ia)

| R | $R^1_n$ | A | R | $R^1_n$ | A |
|---|---|---|---|---|---|
| 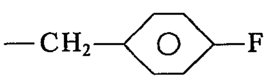 | 7-Cl | N | 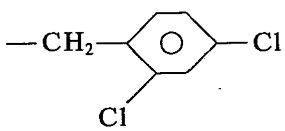 | 7-Cl | N |
| 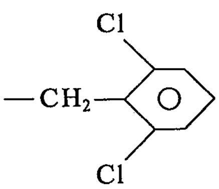 | 7-Cl | N | 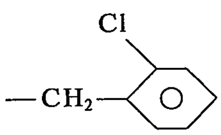 | 7-Cl | N |
| 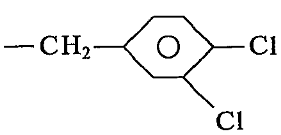 | 7-Cl | N | 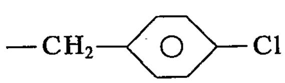 | – | N |
| 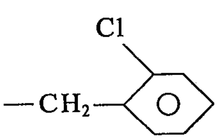 | – | N | 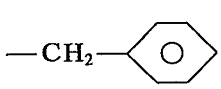 | 7-Cl | CH(N) |
| 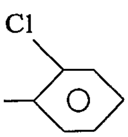 | 7-Cl | CH(N) | 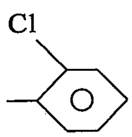 | – | CH(N) |
| 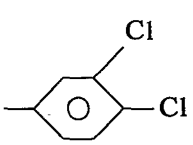 | 7-Cl | CH(N) | 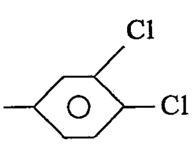 | – | CH(N) |

Fortsetzung

| R | $R^1_n$ | A | R | $R^1_n$ | A |
|---|---|---|---|---|---|
| 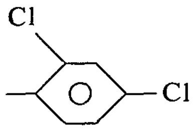 | 7-Cl | CH(N) | 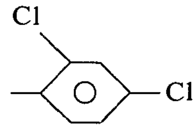 | – | CH(N) |
| 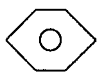 | 7-Cl | CH(N) | 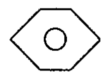 | – | CH(N) |
| 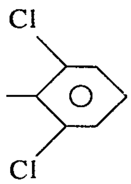 | 7-Cl | CH(N) | 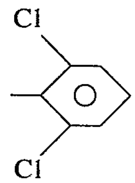 | – | CH(N) |
| 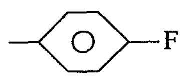 | 7-Cl | CH(N) | 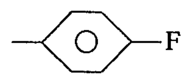 | – | CH(N) |

Verwendet man beispielsweise Fluoren-2-yl-(imidazol-1-yl-methyl)-keton und 4-Chlorphenylmagnesiumchlorid als Ausgangsstoffe, so kann der Reaktionsablauf durch das folgende Formelschema wiedergegeben werden (Verfahren a):

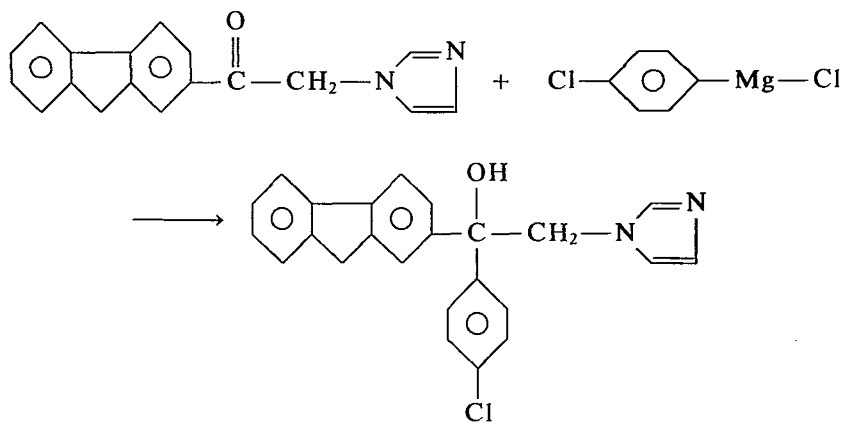

Verwendet man beispielsweise 1-Chlor-2-(7-chlorfluoren-2-yl)-3-(4-chlorphenyl)-propan-2-ol und Imidazol-natrium als Ausgangsstoffe, so kann der Reaktionsablauf durch das folgende Formelschema wiedergegeben werden (Verfahren b):

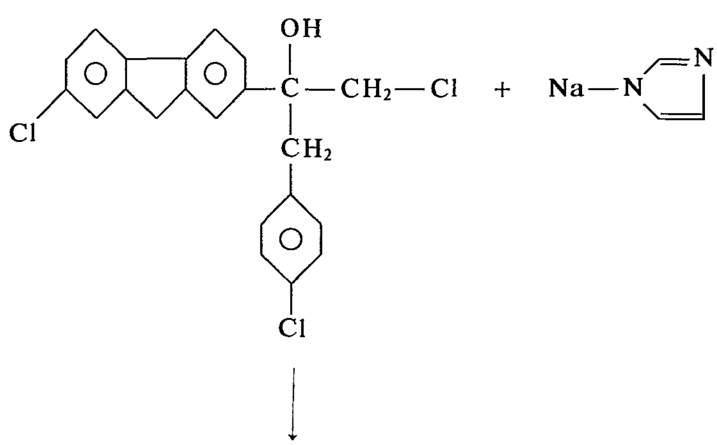

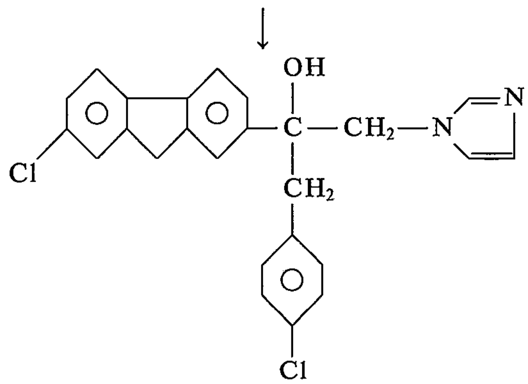

Die für die Verfahrensvariante (a) als Ausgangsstoffe zu verwendenden Fluorenyl-azolylmethyl-ketone sind durch die Formel (II) allgemein definiert.

Die Fluorenyl-azolylmethyl-ketone der Formel (II) sind noch nicht bekannt. Sie lassen sich jedoch in allgemein üblicher und bekannter Weise herstellen, indem man entsprechend Fluorenacylhalogenide der Formel

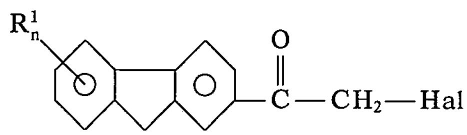

(VI)

in welcher

$R^1$ und n die oben angegebene Bedeutung haben und
Hal für Chlor oder Brom steht,

mit Azolen in Gegenwart eines Verdünnungsmittels, wie beispielsweise Dimethylformamid, und in Gegenwart eines säurebindenden Mittels, wie insbesondere einem Überschuß an Azol, bei Temperaturen zwischen 20 und 80°C umsetzt (vergleiche hierzu auch die Angaben in der US-Patentschrift 3 658 813).

Als Beispiele für die Ausgangsstoffe der Formel (II) seien genannt:

Fluoren-2-yl-(imidazol-1-yl-methyl)-keton
7-Chlorfluoren-2-yl-(imidazol-1-yl-methyl)-keton
7-Fluorfluoren-2-yl-(imidazol-1-yl-methyl)-keton
6-Chlorfluoren-2-yl-(imidazol-1-yl-methyl)-keton
5-Chlorfluoren-2-yl-(imidazol-1-yl-methyl)-keton

Die außerdem für die Verfahrensvariante (a) als Ausgangsstoffe zu verwendenden Grignard-Verbindungen sind durch die Formel (III) allgemein definiert. In dieser Formel steht R vorzugsweise für die Reste, die bei den Verbindungen der Formel (I) bereits vorzugsweise genannt werden.

Die Grignard-Verbindungen der Formel (III) sind allgemein bekannte Verbindungen der organischen Chemie. Als Beispiele seien genannt:

Phenylmagnesiumchlorid, 4-Chlorphenylmagnesiumchlorid, 2,4-Dichlorphenylmagnesiumchlorid, 2,6-Dichlorphenylmagnesiumchlorid, 2-Chlor-6-fluorphenylmagnesiumchlorid, 2-Chlorphenylmagnesiumchlorid, 3-Chlorphenylmagnesiumchlorid, 3,4-Dichlorphenylmagnesiumchlorid, Benzylmagnesiumchlorid, 4-Chlorbenzylmagnesiumchlorid, 2,4-Dichlorbenzylmagnesiumchlorid, 2,6-Dichlorbenzylmagnesiumchlorid, 2-Chlor-6-fluorbenzylmagnesiumchlorid, 2-Chlorbenzylmagnesiumchlorid, 3-Chlorbenzylmagnesiumchlorid, 3,4-Dichlorbenzylmagnesiumchlorid sowie die entsprechenden Bromide.

Die für das Verfahren (b) als Ausgangsstoffe zu verwendenden Fluorenyl-halogenmethyl-carbinole sind durch die Formel (IV) allgemein definiert. In dieser Formel stehen R und $R^1_n$ vorzugsweise für die Reste, die bei den Verbindungen der Formel (I) bereits vorzugsweise genannt wurden.

Die Fluorenyl-halogenmethyl-carbinole der Formel (IV) sind noch nicht bekannt. Sie lassen sich jedoch in allgemein üblicher und bekannter Weise herstellen, indem man Ketone der Formel (VI) mit Grignard-Verbindungen der Formel (III) entsprechend der Verfahrensvariante (a) umsetzt (vergleiche hierzu auch die Angaben in der DE-A-2 623 129 sowie die Herstellungsbeispiele).

Die außerdem für die Verfahrensvariante (b) als Ausgangsstoffe zu verwendenden Azole sind durch die Formel (V) allgemein definiert. In dieser Formel steht Az vorzugsweise für die Reste, die bei den Verbindungen der Formel (I) bereits vorzugsweise genannt wurden und Z steht vorzugsweise für Wasserstoff, Natrium oder Kalium.

Die Azole der Formel (V) sind allgemein bekannte Verbindungen der organischen Chemie.

Für die erfindungsgemäße Umsetzung nach Verfahren (a) kommen als Verdünnungsmittel alle für eine Grignard-Reaktion üblichen Lösungsmittel in Frage. Hierzu gehören vorzugsweise Ether, wie Diethylether oder Tetrahydrofuran sowie Gemischen mit anderen organischen Solventien, wie z. B. Benzol.

Die Reaktionstemperaturen können beim Verfahren (a) in einem größeren Bereich variiert werden. Im allgemeinen arbeitet man zwischen etwa 20 bis etwa 120° C, vorzugsweise zwischen etwa 30 bis etwa 80° C.

Bei der Durchführung des Verfahrens (a) setzt man auf 1 Mol der Verbindung der Formel (II) vorzugsweise einen Überschuß von 3 bis 5 Mol der Grignard-Verbindung der Formel (III) ein. Die Isolierung der Verbindungen der Formel (I) erfolgt in üblicher und bekannter Weise.

Für die erfindungsgemäße Umsetzung nach Verfahren (b) kommen als Verdünnungsmittel vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Benzol, Toluol und Dichlorbenzol; Formamide, wie insbesondere Dimethylformamid; und halogenierte Kohlenwasserstoffe, wie Methylenchlorid, Tetrachlorkohlenstoff oder Chloroform.

Wird das erfindungsgemäße Verfahren (b) in Gegenwart eines Säurebinders vorgenommen, so kann man alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylmethylamin, N,N-Dimethylbenzylamin, weiterhin Pyridin und Diazabicyclooctan. Vorzugsweise verwendet man einen Überschuß an Azol.

Die Reaktionstemperaturen können beim Verfahren (b) in einem größeren Bereich variiert werden. Im allgemeinen arbeitet man zwischen etwa 30 bis 200° C, vorzugsweise bei der Siedetemperatur des Lösungsmittels.

Bei der Durchführung des erfindungsgemäßen Verfahrens (b) setzt man auf 1 Mol der Verbindungen der Formel (IV) vorzugsweise 1 bis 2,5 Mol Azol und 1 bis 2,5 Mol Säurebinder ein. Bei Verwendung eines Alkalisalzes setzt man vorzugsweise auf 1 Mol der Verbindung der Formel (IV) 1 bis 1,5 Mol Alkalisalz ein. Zur Isolierung der Verbindungen der Formel (I) wird das Lösungsmittel abdestilliert, der Rückstand direkt oder nach Aufnahme mit einem organischen Solvens mit Wasser gewaschen, die organische Phase gegebenenfalls über Natriumsulfat getrocknet und im Vakuum vom Lösungsmittel befreit. Der Rückstand wird gegebenenfalls durch Destillation, Umkristallisation oder chromatographisch gereinigt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dematomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie

Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Dentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirksotffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe

enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Beispiel A

### Antimykotische in-vitro-Wirksamkeit

### Versuchsbeschreibung

Die in-Vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze: Sabouraud's milieu d'épreuve
b) für Hefen: Fleischextrakt-Traubenzucker-Bouillon.

Die Bebrütungstemperatur betrug 27° C, die Bebrütungsdauer lag bei 24 bis 96 Stunden.

Die erfindungsgemäßen Verbindungen zeigen bei diesen Tests sehr gute minimale Hemmkonzentrationen und erweisen sich dadurch den bekannten Verbindungen überlegen.

## Beispiel B

### Antimikrobielle in-vivo-Wirksamkeit (oral) bei Mäuse-Candidose

### Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50 – 100 mg/kg Körpergewicht der Präparate oral behandelt.

### Ergebnis

Unbehandelte Tiere starben 3 bis 6 Tage post infektionem. Die Überlebensrate am 6. Tag post infektionem betrug bei unbehandelten Kontrolltieren etwa 5%.

Die bekannten Vergleichsbedingungen zeigten keine Wirkung. Dagegen zeigte die erfindungsgemäße Verbindung des Beispiels 2 eine sehr gute Wirkung ($>$90% Überlebende am 6. Tag p. i.).

## Beispiel C

### Antimykotische in-vivo-Wirksamkeit (oral) am Modell der experimentellen Meerschweinchen-Trichophytie

### Versuchsbeschreibung

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert. Bei unbehandelten Tieren entwickelt sich innerhalb 12 Tagen p. i. das typische Bild

einer Dermatophytose mit Rötung, Schuppung und Haarausfall bis zum totalen Integument-Defekt an der Infektionsstelle. Die infizierten Tiere wurden — beginnend mit dem 3. Tag p. i. — 1mal täglich mit 1%igen Polyethylenglykol-Lösungen der erfindungsgemäßen Präparate lokal behandelt.

Am 14. Tag p. i. zeigten die unbehandelten Kontrolltiere sowie die mit den Vergleichswirkstoffen behandelten das typische Bild einer Dermatophytose, während die Prüfpräparate den Ablauf der Infektion gehemmt hatten.

Die erfindungsgemäßen Verbindungen zeigen bei diesen Tests sehr gute lokale in-vivo-Wirksamkeiten und erwiesen sich dadurch den bekannten Verbindungen überlegen.

## Herstellungsbeispiele

### Beispiel 1

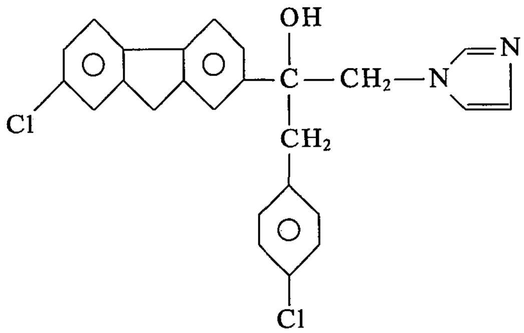

(Verfahren b)

Eine Lösung von 4,2 g (0,078 Mol) Natriummethylat in 22 ml Methanol wird mit 9 g (0,12 Mol) Imidazol versetzt; anschließend wird eine Lösung von 2-(7-Chlorfluoren-2-yl)-3-chlor-1-(4-chlorphenyl)-propan-2-ol in 45 ml Dimethylformamid zugetropft und 90 Minuten auf 60° C erwärmt. Das Gemisch wird in 2000 ml Wasser gegossen, die abgeschiedene kristalline Masse wird in Methylenchlorid gelöst, die Lösung mit Wasser gewaschen, über Natriumsulfat getrocknet, filtriert und im Vakuum eingeengt. Der Rückstand wird durch Verrühren mit Diethylether zur Kristallisation gebracht; die Kristalle werden mit wenig Methylalkohol verrührt und abgesaugt. Man erhält 16,2 g (62,5% der Theorie) 2-(7-Chlorfluoren-2-yl)-1-(4-chlorphenyl)-3-(imidazol-1-yl)-propan-2-ol vom Schmelzpunkt 218° C.

### Herstellung des Ausgangsproduktes

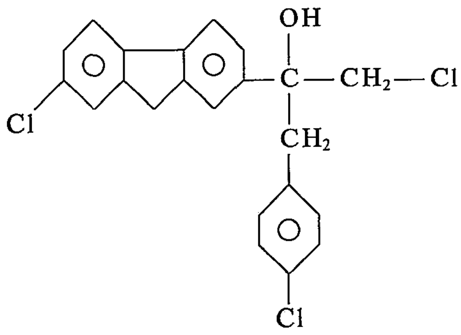

Zu einer Lösung von 4-Chlorbenzylmagnesiumchlorid, erhalten aus 5,4 g (0,22 Mol) Magnesium und 32,2 g (0,2 Mol) 4-Chlorbenzylchlorid in 90 ml Diethylether, werden 27,7 g (0,1 Mol) 2-(7-Chlorfluoren-2-yl)-chlormethylketon portionsweise zugegeben und 1 Stunde nachgerührt. Das Gemisch wird auf Ammoniumchlorid-Lösung gegossen, die abgetrennte Etherphase wird mit Wasser gewaschen, über Natriumsulfat getrocknet. Durch Verrühren mit Diisopropylether wird das erhaltene Öl zur Kristallisation gebracht. Man erhält 24,3 g (61% der Theorie) 2-(7-Chlorfluoren-2-yl)-3-chlor-1-(4-chlorphenyl)-propan-2-ol vom Schmelzpunkt 108° C.

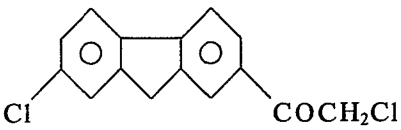

Zu einer Lösung von 401 g (2,0 Mol) 2-Chlorfluoren und 176 ml (2,2 Mol) Chloracetylchlorid in 1000 ml Methylenchlorid werden bei 0°C 293,7 g (2,2 Mol) Aluminiumchlorid portionsweise eingetragen. Nach 1 Stunde wird das Gemisch mit eiskalter verdünnter Salzsäure zersetzt und die organische Phase mit Wasser gewaschen, über Natriumsulfat getrocknet und im Vakuum eingeengt. Die erhaltenen Kristalle werden mit Ethylalkohol verrührt und abgesaugt. Man erhält 426,3 g (77% der Theorie) 2-(7-Chlorfluoren-2-yl)-chlormethyl-keton vom Schmelzpunkt 180°C.

In entsprechender Weise werden sowohl nach Verfahren (a) als auch nach Verfahren (b) die Verbindungen der nachfolgenden Tabelle 1 erhalten.

Tabelle 1

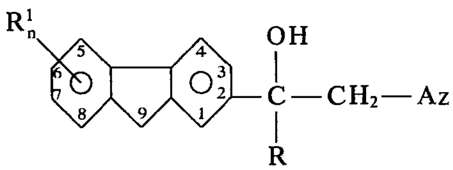

| Bsp. Nr. | R | $R^1_n$ | Az | Schmelzpunkt (°C) |
|---|---|---|---|---|
| 2 | 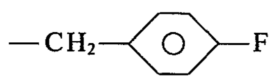 | 7-Cl | Imidazol-1-yl | 210 |
| 3 | 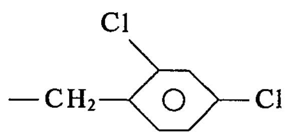 | 7-Cl | Imidazol-1-yl | 222 |
| 4 | 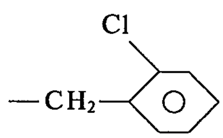 | 7-Cl | Imidazol-1-yl | 214 |
| 5 | 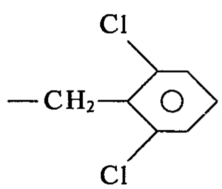 | 7-Cl | Imidazol-1-yl | 140 |
| 6 | 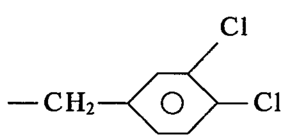 | 7-Cl | Imidazol-1-yl | 135 |
| 7 | 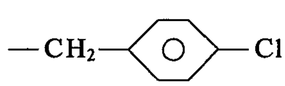 | – | Imidazol-1-yl | 196 |
| 8 | 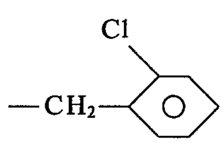 | – | Imidazol-1-yl | 156 |
| 9 | 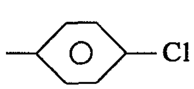 | 7-Cl | Imidazol-1-yl | 242 |

Fortsetzung

| Bsp. Nr. | R | $R^1_n$ | Az | Schmelzpunkt (° C) |
|---|---|---|---|---|
| 10 | 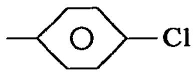 | – | Imidazol-1-yl | 238 |
| 11 | 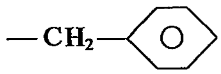 | 7-Cl | Imidazol-1-yl | 255 |
| 12 | 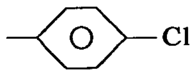 | 7-Cl | 1,2,4-Triazol-1-yl | 186 |
| 13 | 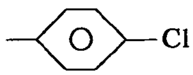 | – | 1,2,4-Triazol-1-yl | |
| 14 | 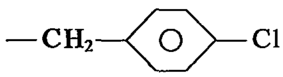 | 7-Cl | 1,2,4-Triazol-1-yl | |
| 15 | 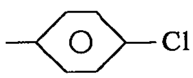 | 7-Cl | 1,3,4-Triazol-1-yl | 214 |

**Patentansprüche**

1. Fluorenyl-azolylmethyl-carbinole der Formel

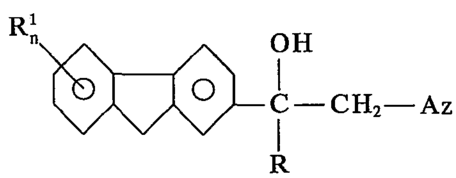

(I)

in welcher

Az für Imidazol-1-yl, 1,2,4-Triazol-1-yl oder 1,3,4-Triazol-1-yl steht,
R für gegebenenfalls durch Fluor oder Chlor, ein- oder zweifach substituiertes Phenyl oder Benzyl steht,
$R^1$ für Fluor oder Chlor steht und
n für 0, 1 oder 2 steht,

und deren physiologisch verträgliche Säureadditions-Salze.

2. Verfahren zur Herstellung von Fluorenyl-azolylmethyl-carbinole der Formel (I) gemäß Anspruch 1, dadurch gekennzeichnet, daß man in an sich bekannter Weise

a) Fluorenyl-azolylmethyl-ketone der Formel

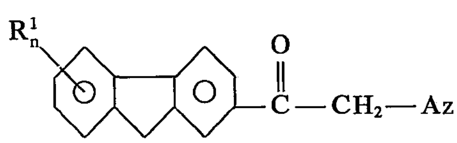

(II)

in welcher

Az, $R^1$ und n die oben angegebene Bedeutung haben,
mit einer Grignard-Verbindung der Formel

$$R-Mg-X \quad (III)$$

in welcher

R die oben angegebene Bedeutung hat und
X für Halogen, insbesondere Chlor oder Brom steht,

in Gegenwart eines Verdünnungsmittels umsetzt, oder

b) Fluorenyl-halogenmethyl-carbinole der Formel

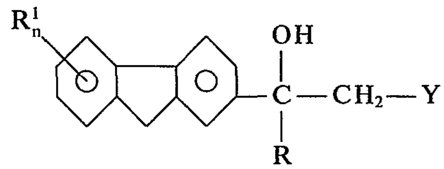

(IV)

in welcher

R, $R^1$ und n die oben angegebene Bedeutung haben und
Y für Halogen, insbesondere Chlor oder Brom steht,

mit Azolen der Formel

$$Z—Az \quad (V)$$

in welcher

Az die oben angegebene Bedeutung hat und
Z für Wasserstoff oder ein Alkalimetall steht,

gegebenenfalls in Gegenwart eines Säurebindemittels und gegebenenfalls in Gegenwart eines Verdünnungsmittels umsetzt und gegebenenfalls die Verbindungen der Formel (I) durch Umsetzen mit Säuren in die Salze überführt.

3. Arzneimittel, gekennzeichnet durch einen Gehalt an mindestens einem Fluorenyl-azolylmethyl-carbinol gemäß Anspruch 1.

4. Verfahren zur Herstellung von antimykotischen Mitteln, dadurch gekennzeichnet, daß man Fluorenylazolylmethylcarbinole gemäß Anspruch 1 mit inerten, nichttoxischen, pharmazeutisch geeigneten Trägerstoffen vermischt.

**Claims**

1. Fluorenyl-azolylmethyl-carbinols of the formula

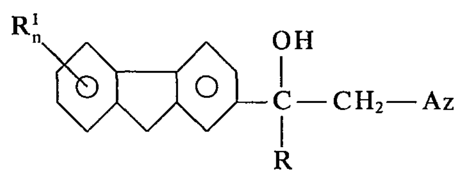

(I)

in which

Az represents imidazol-1-yl, 1,2,4-triazol-1-yl or 1,3,4-triazol-1-yl,
R represents phenyl or benzyl optionally monosubstituted or disubstituted by fluorine or chlorine,
$R^1$ represents fluorine or chlorine and
n represents 0, 1 or 2,

and physiologically acceptable acid addition salts thereof.

2. Process for the preparation of fluorenyl-azolyl-methyl-carbinols of the formula (I) according to Claim 1, characterised in that, in a manner known per se,

a) fluorenyl azolylmethyl ketones of the formula

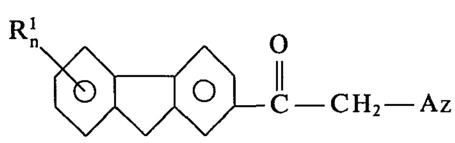

(II)

in which
Az, $R^1$ and n have meaning indicated above,
are reacted with a Grignard compound of the formula

$$R—Mg—X \quad (III)$$

in which

R has the meaning indicated above and
X represents halogen, in particular chlorine or bromine,

in the presence of a diluent, or
b) fluorenyl-halogenomethyl-carbinols of the formula

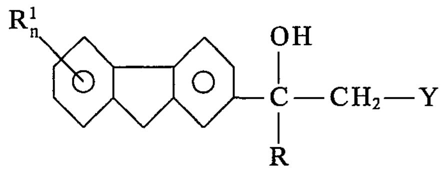

(IV)

in which

R, $R^1$ and n have the meaning indicated above and
Y represents halogen, in particular chlorine or bromine,

are reacted with azoles of the formula

$$Z—Az \quad (V)$$

in which

Az has the meaning indicated above and
Z represents hydrogen or an alkali metal,

if appropriate in the presence of an acid-binding agent and if appropriate in the presence of a diluent, and the compounds of the formula (I) are optionally converted into salts by reaction with acids.

3. Medicaments, characterised in that they contain at least one fluorenyl-azolylmethyl-carbinol according to Claim 1.

4. Process for the preparation of antimycotic agents, characterised in that fluorenyl-azolylmethyl-carbinols according to Claim 1 are mixed with inert, non-toxic, pharmaceutically suitable excipients.

## Revendications

1. Fluorényl-azolylméthyl-carbinols de formule:

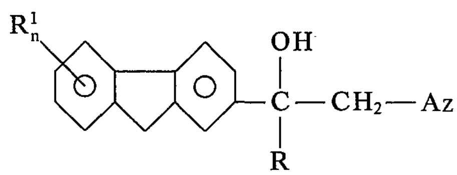

(I)

dans laquelle

Az représente un groupe imidazol-1-yle, un groupe 1,2,4-triazol-1-yle ou un groupe 1,3,4-triazol-1-yle,
R représente un groupe benzyle ou un groupe phényle éventuellement substitué une ou deux fois par un atome de fluor ou par un atome de chlore,
$R^1$ représente un atome de fluor ou un atome de chlore, et
n est égal à 0, 1 ou 2,

ainsi que leurs sels d'addition d'acide physiologiquement acceptables.

2. Procédé de préparation de fluorényl-azolylméthyl-carbinols de formule (I) suivant la revendication 1, caractérisé en ce que, de façon connue en soi:

a) on fait réagir des fluorényl-azolylméthyl-cétones de formule:

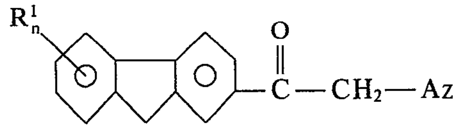

(II)

dans laquelle
Az, $R^1$ et n ont les significations indiquées ci-dessus,
avec un composé de Grignard de formule:

$$R—Mg—X \qquad (III)$$

dans laquelle

R  a la signification indiquée ci-dessus, et
X  représente un atome d'halogène, en particulier, un atome de chlore ou un atome de brome,

en présence d'un diluant, ou
b) on fait réagir des fluorényl-halogènométhyl-carbinols de formule:

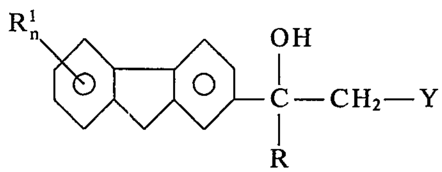

(IV)

dans laquelle

R, $R^1$ et n ont les significations indiquées ci-dessus, et
Y  représente un atome d'halogène, en particulier, un atome de chlore ou un atome de brome,

avec des azols de formule:

$$Z—Az \qquad (V)$$

dans laquelle

Az  a la signification indiquée ci-dessus, et
Z  représente un atome d'hydrogène ou un métal alcalin,

éventuellement en présence d'un agent fixateur d'acide et éventuellement en présence d'un diluant, puis on transforme éventuellement les composés de formule (I) en sels par réaction avec des acides.

3. Médicaments, caractérisés en ce qu'ils contiennent au moins un fluorényl-azolylméthyl-carbinol suivant la revendication 1.

4. Procédé de préparation d'agents antimycotiques, caractérisé en ce qu'on mélange des fluorényl-azolylméthyl-carbinols suivant la revendication 1 avec des substances supports inertes, non toxiques et pharmaceutiquement appropriées.